# EUROPÄISCHE PATENTSCHRIFT

(11) **EP 0 776 230 B1**
(45) Veröffentlichungstag und Bekanntmachung des Hinweises auf die Patenterteilung: **24.10.2001**
(21) Anmeldenummer: 95926829.3
(22) Anmeldetag: 16.08.1995
(51) Int. Cl.: A61M 37/00

(54) **IMPLANTAT UND APPLIKATIONSVORRICHTUNG**
IMPLANT AND DEVICE FOR INSERTING THE IMPLANT
IMPLANT ET DISPOSITF D'IMPLANTATION

(30) Priorität: 17.08.1994 CH 253394
(43) Veröffentlichungstag der Anmeldung: 04.06.1997
(73) Patentinhaber: BOSTON SCIENTIFIC CORPORATION, Natick, MA 01760-1537 (US)
(72) Erfinder: LUESCHER, Patrik, CH-8330 Pfäffikon (CH); WINTERMANTEL, Erich, CH-5442 Fislisbach (CH)
(74) Vertreter: Groner, Manfred
(86) Internationale Anmeldenummer: CH9500184
(87) Internationale Veröffentlichungsnummer: WO9604954

(56) Entgegenhaltungen:
- EP-A- 0 186 632
- WO-A-91/13592
- WO-A-93/00127
- WO-A-94/16632
- DE-A- 3 115 763
- FR-A- 2 696 636
- US-A- 4 237 885
- US-A- 4 402 308
- US-A- 4 820 267
- US-A- 4 994 069

## Beschreibung

Die Erfindung betrifft ein Implantat, eine Vorrichtung und eine Anordnung zum Applizieren eines Implantates.

In der Medizin sind Implantate für viele unterschiedliche Verwendungen und in zahlreichen Ausführungen bekannt. Sie werden in der Regel als ganzes eingesetzt, was einen vergleichsweise grossen operativen Eingriff mit entsprechend hoher Belastung des Patienten bedeutet.

Die US-A-4,994,069 (Ritchard et al) offenbart einen Draht mit einem Formgedächtnis, der in einem Katheter in linearer Form transportiert wird und nach der Abgabe die vorgegebene gebogene Struktur annimmt. Der Draht besitzt Windungen, die eine schraubenförmige Form bilden, wenn er entspannt ist. Er ist dazu bestimmt, in ein Gefäss abgelegt zu werden, um dieses zu verschliessen.

Die FR-A-2 696 636 (Plowiecki) offenbart Spiralen, die in entspanntem Zustand eine Schraubenform besitzen und die zum Verschliessen von Gefässen durch einen Katheter abgebbar sind.

Die WO 93/00127 offenbart eine Vorrichtung mit einer Kanüle für die Abgabe von Medikamenten.

Der Erfindung liegt die Aufgabe zugrunde, ein Implantat zu schaffen, das mit vergleichsweise geringer Belastung des Patienten einsetzbar ist und das sich zudem durch eine breite Anwendung auszeichnet. Mit dem gleichen Zweck ist es Aufgabe der Erfindung, eine Vorrichtung zum Applizieren eines Implantates zu schaffen.

Die Aufgabe ist durch ein Implantat gemäss Anspruch 1 gelöst. Das Fadenknäuel kann in situ durch einen kleinen Einstich und mit geringer Belastung des Patienten in operativer Mikrotechnik und in beliebiger Menge eingebracht werden. Eine sehr breite Palette möglicher Anwendungen ergibt sich insbesondere dadurch, dass die Grösse und die Form des Implantates sehr variabel sind und intraoperativ festgelegt werden können. Zudem sind beispielsweise die Porengrösse und Struktureigenschaften des Implantates durch Modifikation der Materialeigenschaften insbesondere des Fadens variierbar. Der Faden kann Träger von biologisch aktiven Substanzen sein und eignet sich dann insbesondere für die kontrollierte Arzneimittelfreigabe oder die Induktion von Körpergewebe. Zahlreiche Anwendungen sind auch in der Zahnmedizin und in der Veterinärmedizin vorgesehen.

Die erfindungsgemässe Vorrichtung sowie eine Anordnung zum Applizieren eines Implantates ergibt sich durch die Merkmale gemäss Anspruch 22 bzw. 29. Da die erfindungsgemässe Vorrichtung das Implantat in fadenförmiger Form und durch den Kanal geführt abgibt, ist eine Applikation in operativer Mikrotechnik und somit eine minimal invasive Implantation möglich.

Gemäss einer Weiterbildung der erfindungsgemässen Vorrichtung ist mit den genannten Mitteln im Kanal ein Fluidstrom erzeugbar, mit dem der Faden durch den Kanal transportiert wird. Das Fluid kann dazu zusammen mit dem Faden durch die distale Oeffnung des Kanals abgegeben werden und kann beispielsweise als Träger von biologisch aktiven Substanzen oder als Kleber zur örtlichen Stabilisierung des abgegebenen Fadens im Gewebe ausgebildet sein. Denkbar ist jedoch auch eine Ausführung, bei welcher das Fluid über einen rückführenden Kanal weggeführt wird. Das Fluid kann eine Flüssigkeit oder Suspension und insbesondere autologes Blut oder eine Elektrolytlösung, jedoch auch ein Gas sein.

Das Implantat kann in fadenförmiger Form an den Applikationsort gebracht und dort als dreidimensionaler Fadenknäuel abgegeben werden. Die Erfindung ermöglicht die Einführung eines Implantates durch eine bestehende oder eine geschaffene kleine Körperöffnung. Eine Behandlung ist somit mit minimaler Belastung des Patienten möglich. Trotzdem kann das Implantat in der ausgebildeten Form ein grosses Volumen aufweisen, beispielsweise bei einer Anwendung in der Orthopädie, wo das Fadenknäuel eine vergleichsweise grosse Gewebelücke insbesondere einen Knochendefekt ausfüllen kann. Die Länge des Fadens ist durch den behandelnden Arzt genau bestimmbar und damit beispielsweise die Verabreichung eines Medikamentes sehr genau dosierbar.

Nach einer Weiterbildung der Erfindung ist der Faden so einführbar, dass ein Ende des Fadens aus der Einstichstelle bzw. Körperöffnung herausragt. Ein solches Implantat kann jederzeit sehr einfach explantiert werden, indem der Faden am herausragenden Ende gefasst und aus der Einstichstelle herausgezogen wird.

Weitere Merkmale und Vorteile ergeben sich aus den abhängigen Patentansprüchen, der Beschreibung sowie der Zeichnung. Ausführungsbeispiele der Erfindung werden nachfolgend anhand der Zeichnung näher erläutert. Es zeigen:
- Fig. 1: schematisch eine teilweise geschnittene Ansicht einer erfindungsgemässen Vorrichtung,
- Fig. 2 und 3: schematisch das Applizieren eines erfindungsgemässen Implantates,
- Fig. 4: ein in ein Gewebe eingeführtes Implantat,
- Fig. 5: schematisch eine teilweise geschnittene Ansicht einer erfindungsgemässen Vorrichtung gemäss einer Variante,
- Fig. 6: schematisch ein Implantat mit einem an diesem angeschlossenen Injektor, und
- Fig. 7: ein Abschnitt eines Fadens in vergrössertem Massstab.

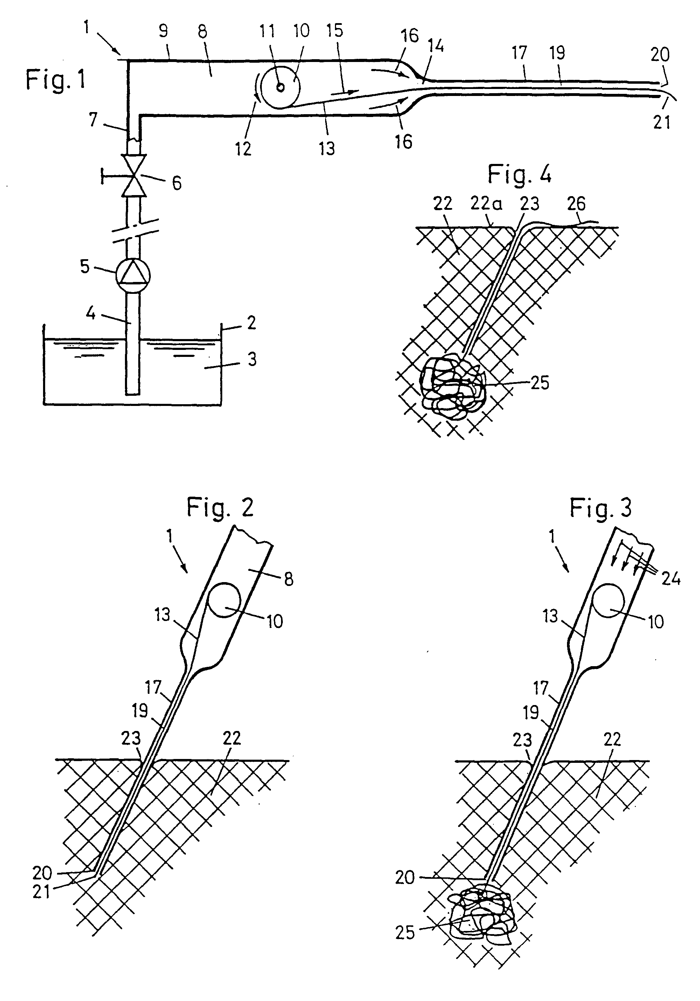

Die erfindungsgemässe Vorrichtung 1 weist gemäss Figur 1 ein Gehäuse 9 auf, welches einen Innenraum 8 besitzt, der in einen Kanal 19 einer Hohlnadel 17 mündet und der an eine Leitung 7 angeschlossen ist, über welche dem Hohlraum 8 aus einem Behälter 2 ein Fluid 3 und insbesondere eine Flüssigkeit zuführbar ist. Das Fluid 3 wird mittels eines Ansaugrohres 4 und einer Pumpe 5 der Leitung 7 zugeführt, in welcher zudem ein Ventil 6 zur Dosierung des Fluidstromes eingesetzt ist.

Im Innenraum 8 ist zudem eine Fadenspule 10 auf einer gehäusefesten Achse 11 in Richtung des Pfeiles 12 drehbar gelagert. Die Spule 10 ist so angeordnet, dass ein auf dieser aufgewickelter Faden 13 in Richtung des Pfeiles 15 in den Kanal 19 der Hohlnadel 17 abspulbar ist. Der Faden 13 ist hierbei in eine proximale Oeffnung 14 des Kanales 19 eingeführt und verlässt den Kanal 19 durch eine distale Oeffnung 20. Denkbar ist auch eine Ausführung, bei welcher die Spule 10 ausserhalb des Gehäuses 9 angeordnet ist. Weiter sind Ausführungen denkbar, bei denen die Spule 10 durch eine andere geeignete Vorratsvorrichtung ersetzt ist. Schliesslich sind Ausführungen denkbar, bei denen der Faden 13 kürzer oder nicht wesentlich länger als der Kanal 19 ist und eine Spule 10 oder dergleichen nicht erforderlich ist. Der Kanal 19 ist so ausgebildet, dass der Faden 13 im wesentlichen ohne Reibung in diesem gleiten kann und zudem Fluid aus dem Innenraum 8 in Richtung der Pfeile 16 in die proximale Oeffnung 14 und in den Kanal 19 einströmen und für den Transport des Fadens 13 im Kanal 19 durchströmen kann. Die Geschwindigkeit des Transportes des Fadens 13 im Kanal 19 kann insbesondere durch eine Erhöhung des Fluiddruckes in der Kammer 8 erhöht werden. Der Transport des Fadens kann schliesslich mit einer Unterbrechung des Fluidstromes am Ventil 6 unterbrochen werden. Schliesslich ist es möglich, ein gesamtes Fadenstück durch die distale Oeffnung 20 nach aussen abzugeben.

Der Kanal 19 der Hohlnadel 17 ist so ausgebildet, dass seine distale Oeffnung 20 gemäss Figur 2 durch eine Einstichöffnung 23 an eine gewünschte Stelle im Gewebe 22 oder an sonst eine Stelle eines zu behandelnden Körpers vorschiebbar ist. Wird nun bei der so positionierten Vorrichtung 1 in den Innenraum 8 durch Umstellung des Ventils 6 unter Druck Fluid 3 eingeleitet, so strömt dieses Fluid in Richtung der Pfeile 24 (Fig. 3) gegen die proximale Oeffnung 14 des Kanals 19 in den Kanal 19 ein und transportiert den Faden 13 unter Abwicklung der Spule 10 gegen die distale Oeffnung 20 und schliesslich nach aussen. Das an der Oeffnung 20 austretende Ende 21 des Fadens 13 erfährt im Gewebe 22 einen Widerstand, so dass die nachfolgenden Fadenabschnitte gebogen und schliesslich zu einem Fadenknäuel 25 abgelegt werden, wie die Figur 3 zeigt. Wesentlich ist hierbei, dass der Faden 13 nahe der Oeffnung 20 im Kanal 19 geführt ist und nach aussen gestossen werden kann.

Als Faden 13 wird hier ein zusammenhängendes Gebilde mit im wesentlichen rundem und im Verhältnis zur Länge sehr kleinem Querschnitt verstanden. Der Faden kann auch ein Hohlfaden und/oder porös, d.h. von innen nach aussen durchlässig sein und ein Arzneimittel enthalten. Besonders geeignete Werkstoffe sind anorganische Gele, beispielsweise auf der Basis von Siliziumoxid oder Calciumphosphat, oder Gele aus synthetischen oder natürlichen Polymeren, beispielsweise Polylactidgel oder Calciumalginatgel. Geeignet sind ebefnalls synthetische Polymere, beispielsweise Polyorthoester, oder natürliche Polymere, beispielsweise Collagen oder Heparin. Weiter sind Anwendungen denkbar, bei denen ein Faden aus autologen Blutbestandteilen, beispielsweise ein Fibrin-Thrombozytenfaden, ein Faden aus resorbierbaren keramischen Fasern beispielsweise Calciumphosphatfasern, ein Metallfaden oder ein Kompositfaden aus mehreren Werkstoffen besonders geeignet sind.

Der Faden 13 sollte so ausgebildet sein, dass er wie oben erläutert zu einem Knäuel 25 biegbar oder faltbar ist. Der Faden weist vorzugsweise über seine ganze Länge den gleichen Durchmesser auf. Denkbar sind jedoch auch Ausführungen, bei denen dieser Durchmesser sich entlang des Fadens gleichmässig oder ungleichmässig ändert.

Vorzugsweise wird mit dem Faden 13 an der distalen Oeffnung 20 gleichzeitig Fluid 3 abgegeben, so dass der Knäuel 25 von eingespritztem Fluid umgeben ist. Bei einem so gebildeten Knäuel 25 können der Faden 13 und das Fluid 3 Träger von biologisch aktiven Substanzen oder Partikeln, beispielsweise Zellen sein. Das Fluid 3 kann jedoch auch ein Kleber, beispielsweise ein Fibrinkleber sein, der die Struktur des Knäuels 25 stabilisiert. Durch geeignete Wahl des Fadens und des Fluides sind somit die Eigenschaften des Knäuels 25 sehr variabel. Weiter kann die Grösse und die Ausbildung des Knäuels 25 durch die Länge des Fadens und die Applikationstechnik variiert werden. Die Form und die Grösse des Fadenknäuels 25 kann somit weitgehend intraoperativ festgelegt werden. Ebenfalls in weiten Grenzen beeinflussbar sind die Porengrösse und die Struktureigenschaften des Knäuels 25. Dies ist insbesondere durch die Wahl der Materialeigenschaften des Fadens 13 und des Fluides 3 sowie durch die Applikationstechnik möglich.

Das Fluid 3 kann flüssig oder gasförmig sein. Wird als Fluid 3 ein Gas gewählt, so ist der Behälter 2 entsprechend als Gasbehälter ausgebildet. Eine Pumpe 5 ist in diesem Fall in der Regel nicht erforderlich. Die Wahl des Fluides 3 richtet sich nach der vorgesehenen Anwendung. Besonders geeignet als Fluid 3 sind autologes Blut, autologes Serum oder Blutfraktionen sowie Elektrolytlösungen. Soll das Fluid 3 den Knäuel 25 stabilisieren, so eignet sich dazu insbesondere ein Fibrinkleber, der aus Blut hergestellt werden kann. Für die Induktion von Gewebe kann das Fluid 3 eine Suspension, beispielsweise aus Knochenpulver oder Mikrospheren oder eine Zellsuspension beispielsweise aus Knochenmarkzellen sein. Ist das Fluid 3 ein Gas, so eignet sich hierbei insbesondere Stickstoff.

Aus den obigen Erläuterungen dürfte klargeworden sein, dass das erfindungsgemässe Implantat in der Medizin und auch in der Veterinärmedizin sehr breit anwendbar ist. Nachfolgend werden einige vorteilhafte Anwendungsmöglichkeiten erläutert.

Eine wesentliche Anwendung des erfindungsgemässen Implantates 25 ist die Induktion von Körpergewebe beim sogenannten Tissue Engineering. Der Faden 13 und/oder das Fluid 3 können Träger von Zellen oder Zellsuspensionen sein, die nach der Bildung des Fadenknäuels 25 neues Gewebe aufbauen oder die Bildung von Gewebe induzieren. Vorgesehen ist insbesondere die Bildung von Knochengewebe bei Knochendefekten oder in Hohlräumen zwischen Endoprothesen und Knochen. Ebenfalls kann bei der Fusion von Wirbeln oder Gelenken sowie in der Zahnheilkunde durch eine Implantation des Knäuels 25 Knochengewebe induziert werden. Weitere Anwendungen bei der Induktion von Gewebe sind die Induktion von Callus bei einem Knochenbruch sowie eine Gewebeinduktion in der plastischen Chirurgie beispielsweise Induktion von Bindegewebe, Knorpelgewebe oder Endothel.

Ausser den obengenannten Anwendungen für die Induktion von Gewebe ist auch eine Freisetzung von systemisch wirkenden Arzneimitteln oder von lokal wirkenden Substanzen möglich. Lokal wirkende Arzneimittel sind insbesondere Antibiotika oder Zytotoxine für die Krebsbehandlung. Das erfindungsgemässe Implantat zeichnet sich hierbei insbesondere durch eine gute Dosierbarkeit der wirkenden Substanzen aus. Auch sehr kleine Substanzmengen können durch die Wahl der Länge des Fadens 13 sehr genau bestimmt werden. Zudem ist es möglich, die Freisetzungskinetik durch die Wahl der Dichte des Knäuels 25 zu bestimmen. Ein dichter Knäuel 25 kann eine wirkende Substanz langsamer abgeben als ein lockerer Knäuel 25. Zudem ist eine mehrstufige Abgabe von Wirkstoffen möglich.

Der Faden 13 kann auch ein Zellträger sein, beispielsweise ein Träger von verkapselten xenogenen Zellen, beispielsweise Langerhanszellen, Nervenzellen oder genetisch veränderte Zellen.

Eine weitere Anwendung des erfindungsgemässen Implantates ist die therapeutische Embolisation beispielsweise von Hämangiomen. Hierbei wird der Faden in ein zentrales Gefäss des Hämangioms eingeführt. Das sehr stark thrombogene Fadenknäuel 25 verstopft dann die Blutversorung des Hämangiom.

Eine weitere Anwendung des erfindungsgemässen Implantates wird in der kontrollierten Applikation von Wirkstoffen auf Schleimhäute gesehen. Ein auf einer Schleimhaut haftender Faden wird dazu mit der erfindungsgemässen Vorrichtung auf die Schleimhaut gebracht und setzt die in ihm enthaltenden Wirkstoffe in die Schleimhaut frei.

Das erfindungsgemässe Implantat ist somit bei wesentlichen Anwendungen nicht lasttragend und metabolisch induktiv.

Der Faden 13 kann so in das Gewebe 23 abgegeben werden, dass er vollständig im Gewebe liegt. Ist hingegen eine Explantation des Fadenknäuels 25 oder eine Injektion oder Infusion von Arzneimitteln vorgesehen, so ist es zweckmässig, das hintere Ende 26 des Fadens 13 gemäss Figur 4 so zu legen, dass es aus der Einstichstelle 23 herausragt. Das Ende 26 kann beispielsweise mit einem hier nicht gezeigten Klebstreifen auf der Gewebeaussenseite 22a fixiert werden. Bei einer Explantation wird der Faden 13 am Ende 26 aus dem Gewebe 22 herausgezogen. Ein den Patienten beeinträchtigender operativer Eingriff ist hierbei nicht erforderlich.

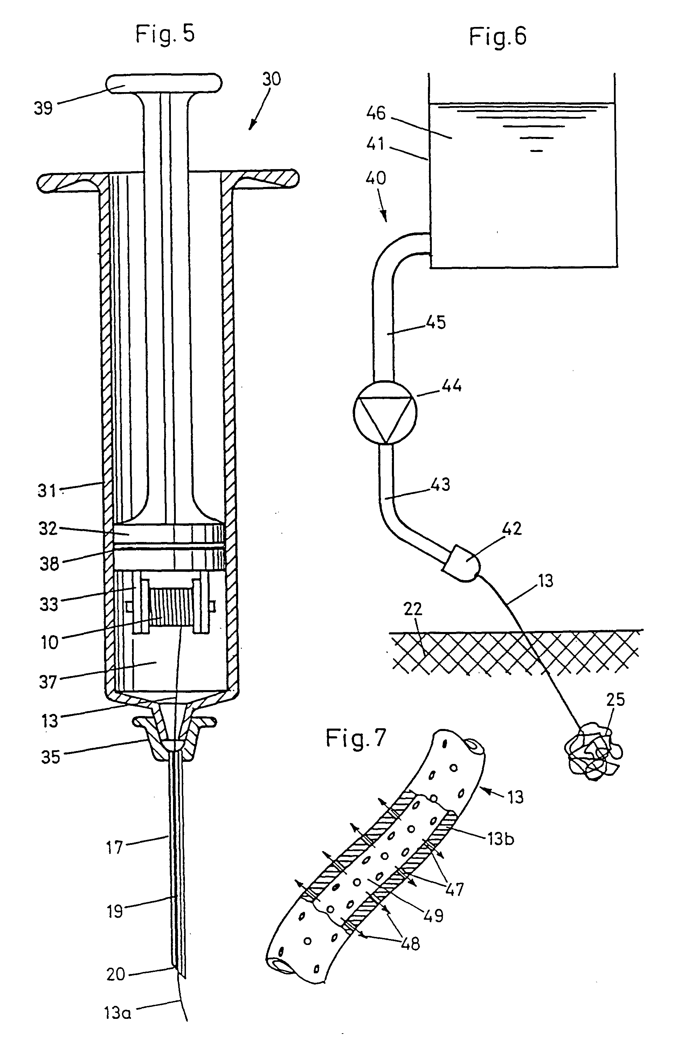

Bei der Ausführung gemäss Figur 5 ist die Vorrichtung zum Applizieren eines Implantates als Spritze 30 ausgebildet. Dies ist insbesondere eine Einwegspritze und weist in einem Gehäuse 31 einen Kolben 32 mit einem Dichtungsring 38 auf. Der Kolben 32 kann an einem Griff 39 in üblicher Weise im Gehäuse 31 verschoben werden und besitzt am vorderen Ende eine Halterung 33 für die Fadenspule 10, auf welcher der Faden 13 aufgespult ist. Der Faden 13 ist vor der. Verwendung der Spritze 30 vorzugsweise bereits mit seinem vorderen Ende 13a wenigstens bereichsweise in den Kanal 19 der Hohlnadel 17 eingeführt. Die Hohlnadel 17 kann wie eine übliche Kanüle ausgebildet und wie an sich bekannt mit einem Aufsteckteil 35 versehen sein. In einem Hohlraum 37 der Spritze 30 befindet sich ein oben erwähntes Fluid. Wird der Kolben 32 in der Anordnung gemäss Figur 5 nach links bewegt, so strömt das Fluid unter entsprechendem Druck in den Kanal 19 und nimmt hierbei den in den Kanal 19 eingeführten Faden 13 mit, der von der frei drehbaren Spule 10 abgespult wird. Am distalen Ende der Hohlnadel 17 wird in einem Gewebe ein Implantat wie oben beschrieben gebildet.

Denkbar sind auch Ausführungen bei denen für den Transport des Fadens 13 im Kanal 19 kein Fluid verwendet wird. Beispielsweise können die Mittel für den Transport eine hier nicht gezeigte angetriebene Rolle aufweisen, welche am distalen Ende der Hohlnadel 17 angeordnet ist und den Faden bewegt.

Ist ein Implantat 25 in einem Gewebe 22 appliziert, so kann an ein, herausragendes Ende des Fadens 13 gemäss Figur 6 mittels eines Adapters 42 ein Injektor 40 angeschlossen werden. Der Injektor 40 weist ein Reservoir 41 mit einem Wirkstoff 46, ein Ansaugrohr 45, eine Pumpe 44 sowie eine Zuleitung 43 auf. Bei laufender Pumpe 44 wird aus dem Reservoir 41 Wirkstoff 46, insbesondere ein Arzneimittel, dem Faden 13 zugeführt. Ist der Faden 13 nun gemäss Figur 7 ein Hohlfaden mit Durchtrittsöffnungen 47 oder porös von innen nach aussen durchlässig, so kann der in den Hohlraum 49 (Fig. 7) des Fadens 13 gelangende Wirkstoff 46 durch die Wandung 13b in Richtung der Pfeile 48 in das Gewebe 22 oder eine Körperöffnung freigesetzt werden. Dadurch ist eine gut dosierbare und gezielte Abgabe eines Wirkstoffes möglich. Das Implantat kann auch in diesem Fall nach der Behandlung entfernt werden.

## Patentansprüche

1. Implantat mit einem Faden (13), der durch eine Hohlnadel (17) implantierbar ist und der entlang seiner Länge zu einer dreidimensionalen, offenporigen Struktur mit einer Mehrzahl von Biegungen biegbar is, wobei der Faden (13) aus einem biegbaren Material hergestellt ist, **dadurch gekennzeichnet, dass** der Faden so biegbar ist, **dass** ein Ende (21) des Fadens (13), das an der Öffnung (20) der Hohlnadel (17) austritt im Körpergewebe (22) einen Widerstand erfährt, so **dass** die nachfolgenden Fadenabschnitte gebogen und schliesslich zu einem Fadenknäuel (25) abgelegt werden.

2. Implantat nach Anspruch 1, **dadurch gekennzeichnet, dass** der Fadenknäuel (25) wenigstens teilweise von einem applizierten Fluid umgeben ist.

3. Implantat nach Anspruch 1 oder 2, **dadurch gekennzeichnet, dass** der Faden (13) des Fadenknäuels (25) und/oder das Fluid (3) Träger von biologisch aktiven Substanzen oder Teilchen, insbesondere Zellen ist.

4. Implantat nach einem der Ansprüche 1-3, **dadurch gekennzeichnet, dass** das Fluid (3) so ausgebildet ist, **dass** es die Struktur oder Form des Fadenknäuels (25) stabilisiert.

5. Implantat nach Anspruch 4, **dadurch gekennzeichnet, dass** das Fluid (3) ein Kleber ist.

6. Implantat nach einem der Ansprüche 1-5, **dadurch gekennzeichnet, dass** es metabolisch induktiv ist.

7. Implantat nach einem der Ansprüche 1-6, **dadurch gekennzeichnet, dass** der Faden (13) des Fadenknäuels (25) aus einem anorganischen Gel oder aus einem Gel aus synthetischen oder natürlichen Polymeren hergestellt ist.

8. Implantat nach Anspruch 7, **dadurch gekennzeichnet, dass** das Gel auf der Basis von Siliziumoxid hergestellt ist oder ein Polylactidgel oder Calciumalginatgel ist.

9. Implantat nach Anspruch 7, **dadurch gekennzeichnet, dass** das Gel auf der Basis von Calciumphosphat hergestellt ist.

10. Implantat nach einem der Ansprüche 1-6, **dadurch gekennzeichnet, dass** der Faden (13) aus einem Polymer oder Polymergel hergestellt ist.

11. Implantat nach Anspruch 10, **dadurch gekennzeichnet, dass** das Polymer ein Collagen, Polysaccharid oder eine Gelatine ist.

12. Implantat nach einem der Ansprüche 1-6, **dadurch gekennzeichnet, dass** der Faden (13) auf der Basis eines Werkstoffes aus autologen Blutbestandteilen hergestellt ist.

13. Implantat nach einem der Ansprüche 1-5, **dadurch gekennzeichnet, dass** der Faden (13) keramische Fasern aufweist.

14. Implantat nach einem der Ansprüche 1-13, **dadurch gekennzeichnet, dass** der Faden (13) hohl und/oder porös ist und insbesondere als Hohlfaden ausgebildet ist.

15. Implantat nach einem der Ansprüche 1-14, **dadurch gekennzeichnet, dass** der Faden als Träger eines Arzneimittels oder Medikaments oder als Träger von Zellen oder einer Zellsuspension ausgebildet ist, und dass der Faden die genannten Mittel nach seiner Implantation als Fadenknäuel abgibt.

16. Implantat nach einem der Ansprüche 1-15, **dadurch gekennzeichnet, dass** der Faden (13) aus einem biologisch abbaubaren Werkstoff hergestellt ist.

17. Implantat nach einem der Ansprüche 2-16, **dadurch gekennzeichnet, dass** das Fluid (3) autologes Blut, autologes Serum oder eine Blutfraktion oder eine Elektrolytlösung aufweist.

18. Implantat nach einem der Ansprüche 2-16, **dadurch gekennzeichnet, dass** das Fluid ein Gas und insbesondere Stickstoff ist.

19. Implantat nach einem der Ansprüche 1-17, **dadurch gekennzeichnet, dass** das Fluid ein biologischer Kleber ist.

20. Implantat nach einem der Ansprüche 2-16, **dadurch gekennzeichnet, dass** das Fluid eine Suspension und insbesondere eine Zellsuspension ist.

21. Implantat nach einem der Ansprüche 1-20, **dadurch gekennzeichnet, dass** der als Fadenknäuel (25) implantierte Faden (13) ein Ende (26) aufweist, das so lang ist, **dass** es aus dem Körper (22) herausragt, in welchem der Fadenknäuel (25) implantiert ist.

22. Vorrichtung zum Applizieren eines Implantates gemäss Anspruch 1, mit einem Kanal (19), in den ein Faden (13) eingeführt ist und der ein proximales und ein in einen Körper einzuführendes distales Ende aufweist und mit Mitteln (10, 3), um den Faden zur Bildung des Implantates durch die distale Öffnung (20) des Kanals (19) abzugeben, **dadurch gekennzeichnet, dass** der Faden aus einem biegbaren Material hergestellt ist, derart, **dass** ein an der Öffnung der Hohlnadel (17) austretendes Ende (21) des Fadens (13) im Gewebe (22) einen Widerstand erfährt, so **dass** die nachfolgenden Fadenabschnitte gebogen und schliesslich zu einen Fadenknäuel (25) abgelegt werden.

23. Vorrichtung nach Anspruch 22, **dadurch gekennzeichnet, dass** mit den Mitteln (10, 3) im Kanal (19) ein diesen durchströmender Fluidstrom erzeugbar ist, derart, **dass** der in den Kanal (19) eingeführte Faden (13) durch den Fluidstrom gegen das distale Ende des Kanals (19) und durch diesen nach aussen bewegt wird.

24. Vorrichtung nach Anspruch 22 oder 23, **dadurch gekennzeichnet, dass** die Mittel (10, 3) eine Fadenspule (10) aufweisen, von welcher der Faden (13) abspulbar ist.

25. Vorrichtung nach Anspruch 23 oder 24, **dadurch gekennzeichnet, dass** das Fluid (3) eine Flüssigkeit, vorzugsweise autologes Blut, autologes Serum oder eine Blutfraktion oder eine Elektrolytlösung ist.

26. Vorrichtung nach Anspruch 23 oder 24, **dadurch gekennzeichnet, dass** das Fluid (3) ein Gas oder ein Gasgemisch und insbesondere Stickstoff ist.

27. Vorrichtung nach Anspruch 23 oder 24, **dadurch gekennzeichnet, dass** das Fluid (3) eine Suspension und insbesondere eine Zellsuspension oder ein Kleber ist.

28. Vorrichtung nach Anspruch 22, **dadurch gekennzeichnet, dass** die Mittel (10, 3) mechanische Transportmittel sind, mit denen ein in den Kanal (19) eingeführter Faden (13) bewegbar ist.

29. Anordnung für die Einführung von biologisch aktiven Substanzen in einen Körper, mit einem Implantat in der Form eines Fadens gemäss Anspruch 1, **gekennzeichnet durch** einen Vorrat an biologisch aktiver Substanz, mit welcher der Faden (13) mit einem Ende in Kontakt ist, um Substanz an das Fadenknäuel (25) abzugeben, wobei der Faden (13) porös ist, derart, dass die Substanz vom Faden (13) abgebbar ist.

30. Anordnung nach Anspruch 29, **gekennzeichnet durch** ein Gehäuse (31), in dem wenigstens ein Teilbereich des Fadens (13) vor der Abgabe des Implantates gespeichert ist.

31. Anordnung nach Anspruch 30, **dadurch gekennzeichnet, dass** an einem distalen Ende des Gehäuses (31) eine Kanüle (17) zur Abgabe des Fadens (13) angebracht ist.

32. Anordnung nach Anspruch 31, **gekennzeichnet durch** einen Flüssigkeitsvorrat, wobei der Faden (13) in der Kanüle (17) infolge eines Flüssigkeitsstromes in der Kanüle (17) zur distalen Öffnung (20) der Kanüle (17) bewegbar ist.

33. Anordnung nach einem der Ansprüche 29 bis 32, **dadurch gekennzeichnet, dass** ein Vorrat der biologisch aktiven Substanz vorgesehen ist und **dass** der Faden (13) mit seinem Ende in Kontakt mit dem Vorrat ist, um biologisch aktive Substanz an das Fadenknäuel (13) abzugeben.

## Claims

1. An implant with a thread (13), which is adapted to be implanted through a hollow needle (17) and which is bendable along its length to form a three-dimensional open-pored structure with a plurality of bends, the thread (13) being made from a bendable material, **characterised in that** the thread is so bendable that one end (21) of the thread (13) which emerges at the opening (20) of the hollow needle (17) experiences a resistance in the body tissue (22) so that the following thread portions are bent and finally deposited in the form of a thread tangle (25).

2. An implant according to claim 1, **characterised in that** the thread tangle (25) is surrounded at least partially by an applied fluid.

3. An implant according to claim 1 or 2, **characterised in that** the thread (13) of the thread tangle (25) and/or the fluid (3) carry biologically active substances or particles, more particularly cells.

4. An implant according to any one of claims 1 to 3, **characterised in that** the fluid (3) is so devised as to stabilise the structure or shape of the thread tangle (25).

5. An implant according to claim 4, **characterised in that** the fluid (3) is an adhesive.

6. An implant according to any one of claims 1 to 5, **characterised in that** it is metabolically inductive.

7. An implant according to any one of claims 1 to 6, **characterised in that** the thread (13) of the tangle (25) is made from an inorganic gel or from a gel of synthetic or natural polymers.

8. An implant according to claim 7, **characterised in that** the gel is made on a silicon oxide basis or is a polyactide gel or calcium alginate gel.

9. An implant according to claim 7, **characterised in that** the gel is made on a calcium phosphate basis.

10. An implant according to any one of claims 1 to 6, **characterised in that** the thread (13) is made from a polymer or polymer gel.

11. An implant according to claim 10, **characterised in that** the polymer is a collagen, polysaccharide or a gelatin.

12. An implant according to any one of claims 1 to 6, **characterised in that** the thread (13) is made on the basis of a material consisting of autologous blood constituents.

13. An implant according to any one of claims 1 to 5, **characterised in that** the thread (13) comprises ceramic fibres.

14. An implant according to any one of claims 1 to 13, **characterised in that** the thread (13) is hollow and/or porous and in particular is made as a hollow thread.

15. An implant according to any one of claims 1 to 14, **characterised in that** the thread is constructed as a carrier for a drug or medicament or as a carrier for cells or a cell suspension, and **in that** the thread delivers the said agents after its implantation in the form of a thread tangle.

16. An implant according to any one of claims 1 to 15, **characterised in that** the thread (13) is made from a biologically degradable material.

17. An implant according to any one of claims 2 to 16, **characterised in that** the fluid (3) comprises autologous blood, autologous serum or a blood fraction or an electrolyte solution.

18. An implant according to any one of claims 2 to 16, **characterised in that** the fluid is a gas and, in particular, nitrogen.

19. An implant according to any one of claims 1 to 17, **characterised in that** the fluid is a biological adhesive.

20. An implant according to any one of claims 2 to 16, **characterised in that** the fluid is a suspension and, in particular, a cell suspension.

21. An implant according to any one of claims 1 to 20, **characterised in that** the thread (13) implanted in the form of a thread tangle (25) has an end (26) of a length such as to project from the body (22) in which the thread tangle (25) is implanted.

22. Device for applying an implant according to claim 1, with a duct (19) into which a thread (13) is introduced and which has a proximal end and a distal end for introduction into a body, and with means (10, 3) to deliver the thread for forming the implant through the distal opening (20) of the duct (19), **characterised in that** the thread is made from a bendable material such that an end (21) of the thread (13) emerging at the opening of the hollow needle (17) experiences a resistance in the tissue (22) so that the following thread portions are bent and finally deposited in the form of a thread tangle (25).

23. A device according to claim 22, **characterised in that** a fluid flow through the duct (19) can be produced with the means (10, 3) in the duct in such manner that the thread (13) introduced into the duct (19) is moved by the fluid flow against the distal end of the duct (19) and out through the latter.

24. A device according to claim 22 or 23, **characterised in that** the means (10, 3) comprise a thread reel (10) from which the thread (13) can be unwound.

25. A device according to claim 23 or 24, **characterised in that** the fluid (3) is a liquid, preferably autologous blood, autologous serum or a blood fraction or an electrolyte solution.

26. A device according to claim 23 or 24, **characterised in that** the fluid (3) is a gas or a gas mixture and, in particular, nitrogen.

27. A device according to claim 23 or 24, **characterised in that** the fluid (3) is a suspension and, in particular, a cell suspension or an adhesive.

28. A device according to claim 22, **characterised in that** the means (10, 3) are mechanical transport means with the aid of which a thread (13) introduced into the duct (19) is movable.

29. An arrangement for introducing biologically active substances into a body, with an implant in the form of a thread according to claim 1, **characterised by** a supply of biologically active substance, with which one end of the thread (13) is in contact in order to deliver substance to the thread tangle (25), the thread (13) being porous in such manner that the substance can be delivered by the thread (13).

30. An arrangement according to claim 29, **characterised by** a housing (31) in which at least a partial zone of the thread (13) is stored before delivery of the implant.

31. An arrangement according to claim 30, **characterised in that** a cannula (17) for delivering the thread (13) is mounted at a distal end of the housing (31).

32. An arrangement according to claim 31, **characterised by** a liquid supply, the thread (13) being movable in the cannula (17) towards the distal opening (20) of the cannula (17) as a result of a flow of liquid in the cannula (17).

33. An arrangement according to any one of claims 29 to 32, **characterised in that** a supply of the biologically active substance is provided and **in that** the thread (13) is in contact by its end with the supply in order to deliver biologically active substance to the thread tangle.

## Revendications

1. Implant avec un fil (13), qui peut être implanté à travers une aiguille creuse (13) et qui peut être plié, le long de sa longueur, en une structure tridimensionnelle à pores ouverts avec un grand nombre de courbures, le fil (13) étant réalisé dans une matière flexible, **caractérisé en ce que** le fil est suffisamment flexible pour qu'une extrémité (21) du fil (13), qui sort de l'ouverture (20) de l'aiguille creuse (17), subisse dans le tissu corporel (22) une résistance, ce qui fait que les portions de fil suivantes sont courbées et finalement repliées en une pelote de fil (25).

2. Implant selon la revendication 1, **caractérisé en ce que** la pelote de fil (25) est entourée au moins partiellement par un fluide appliqué.

3. Implant selon la revendication 1 ou 2, **caractérisé en ce que** le fil (13) de la pelote de fil (25) et/ou le fluide (3) sont porteurs de substances ou de particules actives biologiquement, en particulier de cellules.

4. Implant selon l'une des revendications 1 à 3, **caractérisé en ce que** le fluide (3) est tel qu'il stabilise la structure ou la forme de la pelote de fil (25).

5. Implant selon la revendication 4, **caractérisé en ce que** le fluide (3) est une colle.

6. Implant selon l'une des revendications 1 à 5, **caractérisé en ce qu'**il est inductif métaboliquement.

7. Implant selon l'une des revendications 1 à 6, **caractérisé en ce que** le fil (13) de la pelote de fil (25) est réalisé à partir d'un gel non organique ou à partir d'un gel de polymères synthétiques ou naturels.

8. Implant selon la revendication 7, **caractérisé en ce que** le gel est réalisé à base d'oxyde de silicium ou est un polyactigel ou un gel d'alginate de calcium.

9. Implant selon la revendication 7, **caractérisé en ce que** le gel est réalisé à base de phosphate de calcium.

10. Implant selon l'une des revendications 1 à 6, **caractérisé en ce que** le fil (13) est réalisé à partir d'un polymère ou d'un gel polymère.

11. Implant selon la revendication 10, **caractérisé en ce que** le polymère est un collagène, un polysaccharide ou une gélatine.

12. Implant selon l'une des revendications 1 à 6, **caractérisé en ce que** le fil (13) est réalisé à base d'une matière faite de constituants sanguins autologues.

13. Implant selon l'une des revendications 1 à 5, **caractérisé en ce que** le fil (13) comporte des fibres céramiques.

14. Implant selon l'une des revendications 1 à 13, **caractérisé en ce que** le fil (13) est creux et/ou poreux et réalisé notamment sous la forme d'un fil creux.

15. Implant selon l'une des revendications 1 à 14, **caractérisé en ce que** le fil est réalisé en tant que support d'une substance médicale ou médicament ou en tant que support de cellules ou sous la forme d'une suspension de cellules, et **en ce que** le fil délivre les substances citées après son implantation entant que pelote de fil.

16. Implant selon l'une des revendications 1 à 15, **caractérisé en ce que** le fil (13) est réalisé en une matière biodégradable.

17. Implant selon l'une des revendications 2 à 16, **caractérisé en ce que** le fluide (3) est du sang autologue, un sérum autologue ou une fraction sanguine ou une solution électrolytique.

18. Implant selon l'une des revendications 2 à 16, **caractérisé en ce que** le fluide est un gaz et notamment de l'azote.

19. Implant selon l'une des revendications 1 à 17, **caractérisé en ce que** le fluide est une colle biologique.

20. Implant selon l'une des revendications 2 à 16, **caractérisé en ce que** le fluide est une suspension et notamment une suspension de cellules.

21. Implant selon l'une des revendications 1 à 20, **caractérisé en ce que** le fil (13), implanté sous la forme d'une pelote de fil (25), présente une extrémité (26) qui est suffisamment longue pour ressortir du corps (22) dans lequel la pelote de fil (25) est implantée.

22. Dispositif pour appliquer un implant selon la revendication 1, comportant un canal (19) dans lequel est introduit un fil (13) et qui présente une extrémité proximale et une extrémité distale à introduire dans un corps, et comportant des moyens (10, 3) pour distribuer le fil, destiné à former l'implant, à travers l'ouverture distale (20) du canal (19), **caractérisé en ce que** le fil est constitué d'une matière flexible telle qu'une extrémité (12) du fil (13), se trouvant à l'ouverture de l'aiguille creuse (17), subit dans le tissu (22) une résistance, ce qui fait que les portions de fil suivantes sont courbées et finalement repliées en une pelote de fil (25).

23. Dispositif selon la revendication 22, **caractérisé en ce qu'**avec les moyens (10, 3) on peut produire dans le canal (19) un courant de fluide traversant ce dernier, de manière que le fil (13) introduit dans le canal (19) soit déplacé par le courant de fluide, vers l'extrémité distale du canal (19) et à travers celle-ci, vers l'extérieur.

24. Dispositif selon la revendication 22 ou 23, **caractérisé en ce que** les moyens (10, 3) comportent une bobine de fil (10) de laquelle le fil (13) peut être dévidé.

25. Dispositif selon la revendication 23 ou 24, **caractérisé en ce que** le fluide (3) est un liquide, de préférence du sang autologue, du sérum autologue ou une fraction sanguine ou une solution électrolytique.

26. Dispositif selon la revendication 23 ou 24, **caractérisé en ce que** le fluide (3) est un gaz ou un mélange de gaz, et en particulier de l'azote.

27. Dispositif selon la revendication 23 ou 24, **caractérisé en ce que** le fluide (3) est une suspension et en particulier une suspension de cellules ou une colle.

28. Dispositif selon la revendication 22, **caractérisé en ce que** les moyens (10, 3) sont des moyens de transport mécaniques au moyen desquels un fil (13) introduit dans le canal (19) peut être déplacé.

29. Dispositif pour l'introduction de substances actives biologiquement dans un corps, comportant un implant sous la forme d'un fil selon la revendication 1, **caractérisé par** une réserve d'une substance active biologiquement avec laquelle le fil (13) est en contact par une extrémité, afin de délivrer la substance à la pelote de fil (25), le fil (13) étant poreux de manière que la substance puisse être délivrée par le fil (13).

30. Dispositif selon la revendication 29, **caractérisé par** un boîtier (31) dans lequel est stocké au moins une zone partielle du fil (13) avant que l'implant ne soit délivré.

31. Dispositif selon la revendication 30, **caractérisé en ce qu'**une canule (17) est placée à une extrémité distale du boîtier (31), pour délivrer le fil (13).

32. Dispositif selon la revendication 31, **caractérisé par** une réserve de liquide, le fil (13) étant déplaçable dans la canule (17) sous l'effet d'un courant de liquide dans la canule (17), vers l'ouverture distale (20) de la canule (17).

33. Dispositif selon l'une des revendications 29 à 32, **caractérisé en ce qu'**il est prévu une réserve de la substance active biologiquement et **en ce que** le fil (13) est en contact avec la réserve, par son extrémité, afin de délivrer la substance active biologiquement à la pelote de fil (13).
